# EUROPEAN PATENT SPECIFICATION

(11) **EP 3 716 771 B1**
(45) Date of publication and mention of the grant of the patent: **30.12.2020**
(21) Application number: 19727433.5
(22) Date of filing: 04.06.2019
(51) Int. Cl.: A22B 5/00, A22C 17/00, G01N 33/02, G01N 33/12

(54) **A METHOD OF PROVIDING FEEDBACK DATA INDICATING QUALITY OF FOOD PROCESSING PERFORMED BY AN OPERATOR**
VERFAHREN ZUR BEREITSTELLUNG VON RÜCKMELDEDATEN, DIE DIE QUALITÄT DER VON EINEM BEDIENER DURCHGEFÜHRTEN LEBENSMITTELVERARBEITUNG ANZEIGEN
PROCÉDÉ PERMETTANT DE FOURNIR DES DONNÉES DE RÉTROACTION INDIQUANT LA QUALITÉ DE LA TRANSFORMATION ALIMENTAIRE EFFECTUÉE PAR UN OPÉRATEUR

(30) Priority: 06.06.2018 EP 18176282
(43) Date of publication of application: 07.10.2020
(73) Proprietor: Marel Iceland EHF, 210 Gardabaer (IS)
(72) Inventor: SIGURDSSON, Bjarni Sigthor, 221 Hafnarfjördur (IS); THORODDSEN, Gunnar Atli, 107 Reykjavik (IS); ADALSTEINSSON, Sigurdur Halldor, 112 Reykjavik (IS); GUDJONSDOTTIR, Stella, 101 Reykjavik (IS); THORSTEINSSON, Thomas, 200 Kopavogi (IS); EIDSSON, Thröstur Snaer, 200 Kopavogur (IS)
(74) Representative: Inspicos P/S
(86) International application number: PCT/EP2019/064541
(87) International publication number: WO 2019/234054

(56) References cited:
- EP-A2- 0 361 625
- WO-A1-2008/102148
- WO-A1-2011/095998
- WO-A1-2017/174768
- US-A- 5 428 657
- US-A1- 2003 098 409

## Description

### FIELD OF THE INVENTION

The present invention relates to method and a system for providing feedback data indicating quality of food processing performed by an operator.

### BACKGROUND OF THE INVENTION

Manually processing food products such as fish fillets is a challenging task, especially for food products where it may be difficult to visually detect undesired objects such as bones and cartilages.

In processing plants today, operators that process food products are provided with limited, if any, feedback about the quality of the processing. In fish factories as an example, it may be that the processing plant gets feedback from a customer that that quality of the overall food processing was not good enough.

In some cases, such fish factories may utilize X-ray apparatus to make quality checks to see if e.g. all bones were removed or not. If not, the fish fillets are rejected and typically recirculated into the system. However, individual operators will not be notified about whether or not improvement in the processing is needed.

WO 2011/095998 A1 relates to a food processing apparatus for detecting and cutting tough tissues from food item pieces such as fish or meat fillets or poultry items. At least one x-ray machine is associated to a first conveyor for imaging incoming food items conveyed on the first conveyor and based on the imaging generating x - ray image data indicating the location of the tough tissues in the food item pieces. A vision system supplies second image data of the food item pieces subsequent to the imaging by the at least one x-ray machine. The second image data including position related data indicating the position of the food item pieces on the second conveyor prior to the cutting. A mapping mechanism including a processor determines an estimated coordinate position of the food item pieces on the second conveyor by utilizing the x-ray image data and tracking position data from the tracking mechanism. The processor compares the estimated coordinate position of the food item pieces to the actual position on the second conveyor based on the second image data.

EP 0 361 625 A2 presents a method for examining food products for undesired ingredients by means of irradiation. This method applies a laser beam which scans the food products according to a predetermined pattern. Variations in the local intensity of the laser beam shining through the food products then indicate the presence of undesired ingredients. This method can be carried out by an apparatus which preferably comprises two parabolic mirrors, a laser emitting a laser beam such as to originate from the focus of one of said mirrors and a detection mean positioned in the focus of the other mirror. The food products are moved between the mirrors, for example by a conveyor belt.

WO 2008/102148 A1 describes a method of handling food pieces, comprises subjecting a food piece to an X-ray analysis at an analysis position to detect a foreign object in the food piece. If a foreign object is detected, the food piece is transported from the analysis position to an operator and the operator is provided with an indication of the location of the foreign object in the food piece, characterised in that the step of providing an indication comprises providing a mark on the food piece

US 5 428 657 A describes a method and apparatus for identifying and pinpointing the location of unwanted pieces of material or defects in, for example, de-boned poultry pieces. The poultry pieces to be inspected are carried on a conveyor and passed under an impinging collimated X-ray beam. The Rayleigh scattering resulting is detected and measured, as is the Compton back scattering and the data is processed in a processing unit to determine the location and type of foreign matter involved. The ratio of the Rayleigh and Compton scattering is also determined and used to verify the identity of the foreign material. Transmitted X-rays, i.e., radioscopy, are used to normalize the data, and to aid in a pinpointing of the location of the unwanted material.

### SUMMARY OF THE INVENTION

On the above background it is an object of embodiments of the present invention to provide a method according to claim 1 and a system according to claim 11 which is capable of providing feedback data indicating the quality of the food processing.

In general, the invention preferably seeks to mitigate, alleviate or eliminate one or more of the above mentioned disadvantages of the prior art singly or in any combination. In particular, it may be seen as an object of embodiments of the present invention to provide a feedback method and a system that solves the above mentioned problems, or other problems.

To better address one or more of these concerns, in a first aspect of the invention, a method is provided for providing feedback data indicating quality of food processing, comprising:
- acquiring at least one image data of a processed food product (407) from an operator,
- processing the acquired image data, the processing including:
   ∘ detecting whether undesired objects are present in the processed food product,
   ∘ obtaining, in case undesired objects are detected, position data of the undesired objects within the processed food product,
- utilizing the position data in issuing a feedback indicator indicating a position where said detected undesired objects are present within the food product processed by said operator,

characterized in that in case undesired objects are detected in a subsequent processed food product processed by said operator, where the undesired objects are positioned within the same or similar position as for said processed food product, an intensifier is triggered indicating an increase in magnitude of said feedback indicator.

Accordingly, a method is provided where it is now possible to indicate feedback data about the quality of the food processing. This means that an operator as an example may be directly informed about where improvements are needed in the food processing. More importantly, the operator may be informed where within the food object such improvements are needed which facilitates the improvement for the operator greatly. If the food object is e.g. a fish fillet, the operator may be informed that e.g. within the pin bone area of the fish fillets, improvements are needed in order to remove all the pin bones.

The feedback data may also be issued to e.g. a plant manager so as to allow the plant manager to have an overview over quality of the food processing for all or individual operators.

The term undesired objects may according to the present invention be understood as any objects that are not desirable toward consumers, such as, but not limited to, bones, cartilages, undesired surface tissues.

The food object may be selected from, but is not limited to, fish fillets, poultry meat, beef, pork, lamb.

The acquiring of at least one image data may particularly comprise the step of obtaining an electronic data file representing the image, e.g. obtained by X-ray, laser scanner, or digital camera.

The processing may particularly comprise the step of reading the electronic data file into a computer processing unit and using the computer processing unit and a predefined computer program to detect the undesired objects and to determine the position data.

The feedback indicator may be generated by the computer processing unit.

In one embodiment, said feedback data is visually shown to the operator via a display. In an embodiment, this may visually be shown within outlines of a food product being processed. As an example, if the food product is fish fillets, outlines of a fish fillet may be shown on the screen and the feedback indicator may be illustrated within the correct area within the outlines of the fish fillet so as to allow the operator to immediately know where he/she needs to improve the processing. An image of the actual, or similar, food product that was processed, e.g. said fish fillet, may also be shown with said feedback indicator instead of said outlines.

According to the invention, in case undesired objects are detected in a subsequent processed food product processed by said operator, where the undesired objects are positioned within the same or similar position as for said processed food product, an intensifier is triggered indicating an increase in magnitude of said feedback indicator. In that way, it is possible to indicate repetitive failures in the food processing. Referring to said example, if the operator again in a subsequent fish fillet does not remove all the pin bones, the operator (or e.g. said plant manager) is informed about repetitive failures in that area. Based thereon, the plant manager may e.g. be able to plan additional training for this operator and other operators.

The term "subsequent processed food product" may, in one embodiment, be defined such that two products either directly one after the other in a row of food products or within a certain distance in place or time after the other has the same error. By same error is meant same kind of undesired object or an undesired object in the same location or substantially in the same location.

"Within a certain distance in place" may indicate a number of other food products which can be between the two food products having the same error. In one example, the number may be zero indicating that the magnitude is increased when two directly adjacent food products have the same error. In another example, the number is 10 indicating that the magnitude is increased when two products in a row of ten products have the same error.

"Within a certain distance in time" may indicate a duration in which two food products are experienced with the same error. In one example, the duration may be 30 seconds indicating that the magnitude is increased when two food products have the same error in a period of 30 seconds. In another example, the duration is 2 minutes indicating that the magnitude is increased when two products within a period of 2 minutes have the same error.

In one embodiment, said feedback indicator is utilized as a pixel command in determining a pixel illumination area within a pixel array. Referring to said example, said feedback indicator may be indicated by means of illuminating/altering a portion of the displayed image, e.g. red light, a circle or something similar within said obtained position where the quality of the food processing was lacking. In an embodiment, said intensifier is utilized as a magnification/intensification of said pixel command indicating an enlargement of said illumination area within said pixel array. Referring to previous example, this may be visualized by enlarging and/or intensifying said illuminated region, e.g. enlarging said circle within the pin bone area.

In one embodiment, in case no undesired objects are detected within the same or similar position as for said processed food product in a subsequent processed food product processed by said operator, a de-intensifier is triggered indicating a decrement of said feedback. In that way, the operator or the plant manager may in a positive way be informed that improvement is taking place in the food processing for this particular operator. In an embodiment, said de-intensifier is utilized as a de-magnification of said pixel command indicating a decrement of said illumination area within said pixel array. Referring to said example, this may be performed by visually decreasing the circle therefore immediately informing e.g. the operator that improvement is taking place.

In one embodiment, said at least one acquired image data comprises image data acquired from one or more of the following: X-ray data acquired from an X-ray apparatus, 2 Dimensional (2D) digital data acquired from a 2D digital device, 3D digital data acquired form a 3D digital device. This should however not be construed as being limited to said image device, other image device well known to a person skilled in the art may of course also be implemented.

In one embodiment, the method further comprises associating Identification Data (ID) of said operator to said feedback indicator. In that way, it is possible for e.g. a plant manager to monitor individual operators over time and get a processing history for the individual operators. This data may e.g. then be utilized to evaluate the level of training that individual operators need.

A typical implementation of said method is within a processing plant where incoming food products are received by an infeed conveyor to individual operators arranged along the infeed conveyor. The operators have login ID to the processing stations so that all processed food products are associated to the individual operators. The food products are automatically assigned to individual operators vie e.g. sweep arms. The food products are processed at the processing stations, released to a take away conveyor (which may be the same as the infeed conveyor). After that the processed food products are conveyed through a detection apparatus that acquires said image data, where the acquired image data are processed as already discussed.

According to a second aspect of the invention, an automatic feedback system is provided configured to provide a feedback data indicating quality of a food processing performed by an operator, comprising:
- a detection apparatus for acquiring image data of a processed food product from an operator,
- a processor for processing the acquired image data, the processing including:
   ∘ detecting whether undesired objects are present in the processed food product,
   ∘ determining, in case undesired objects are detected, the position of the undesired objects within the processed food product,

wherein the processor is further configured to utilize the position data in issuing a feedback indicator indicating a position where said detected undesired objects are present within the food product processed by said operator,

characterized in that the processor is configured to identify a subsequent erroneously processed food product processed by said operator, wherein the subsequent erroneously processed food product is a food product with an undesired objects positioned within the same or similar position as for said processed food product.

A feedback system is thus provided capable of giving an operator or e.g. a plant manager valuable feedback indicating the quality of the food processing for individual operators within a food processing plant.

Detection of erroneously processed food product from the same operator could support the operator or a supervisor e.g. relative to need for education etc. Herein, the subsequent erroneously processed food product is defined as a food product with an undesired objects positioned within the same or similar position as for said processed food product. Accordingly, the processor is capable of identifying a food product with an undesired object from a specific operator and to identify a subsequent food product from the same operator.

If such a subsequent erroneously processed food product is identified, the processor may generate an intensifier signal. The intensifier signal could be a visual indication, e.g. a light signal, a tactile indication, such as a vibration signal, or an electronic message, e.g. in the form of a text string or similar data string. The intensifier signal makes it possible for the operator or others to identify that a subsequent erroneously processed food product was identified.

The processor may be configured to determine a distance between the food product with an undesired object and a subsequent erroneously processed food product. The distance may indicate how urgent action is needed. If the subsequent erroneously processed food product is very seldom, i.e. if the distance is large, it may not be urgent to react, whereas if the distance is short, urgency may be required.

The processor may e.g. be configured to determine the distance in a number of separating food products. The separating food product is a food product arranged in a row of food products between the food products with an undesired object and the subsequent erroneously processed food product.

In one example, the processor may be pre-programmable for a specific number of separating food products. As an example, the processor may be pre-programmed for 15 separating food products. In that case, if a food product with an undesired object is determined, and after further processing of 20 food products, a subsequent erroneously processed food product is determined, no intensifier signal is generated. If a food product with an undesired object is determined, and after further processing of 12 food products, a subsequent erroneously processed food product is determined, an intensifier signal is generated.

The processor may also be configured to determine the distance in the form of a time interval which separates the food product with an undesired object from the subsequent erroneously processed food product.

In one example, the processor may be pre-programmable for a time interval. As an example, the processor may be pre-programmed for 15 minutes. In that case, if a food product with an undesired object is determined, and after further processing of food products in a time interval of 20 minutes, a subsequent erroneously processed food product is determined, no intensifier signal is generated. If a food product with an undesired object is determined, and after further processing of food products in a time interval of 12 minutes, a subsequent erroneously processed food product is determined, an intensifier signal is generated

In one embodiment, said system further comprises a feedback device associated to the operator, where the feedback device is operably connected to a processor (or said processor) and comprises a display, where said feedback indicator is utilized as a pixel command in determining a pixel illumination area within the display. In that way, the operator or e.g. said plant manager may in a very user friendly way be informed where the food processing needs improvements.

The intensifier signal may particularly be constituted by a change in intensity of the pixel illumination, or a change in size of the illuminated area, or a change in color of the illuminated area.

In one embodiment, said detection apparatus comprises one or more of, but not limited to, an X-ray apparatus and wherein said image data comprises X-ray data, imaging device for acquiring 2D and/or 3D image data.

In one embodiment, said food product is processed at a processing station selected from a plurality of processing stations arranged along an infeed conveyor from which the food product is received, and wherein after processing said food product, it is received by a take-away conveyor and conveyed to said detection apparatus where said image data is acquired. Said infeed conveyor and take-away conveyor may in one embodiment be one and the same conveyor.

In general the various aspects of the invention may be combined and coupled in any way possible within the scope of the invention. These and other aspects, features and/or advantages of the invention will be apparent from and elucidated with reference to the embodiments described hereinafter.

### BRIEF DESCRIPTION OF THE DRAWINGS

Embodiments of the invention will be described, by way of example only, with reference to the drawings, in which
Figure 1 shows a flowchart of a method according to the present invention of providing a feedback data indicating quality of a food processing performed by an operator, Figures 2 and 3 illustrate one embodiment of issuing a feedback indicator, and
Figure 4 shows a system according to the present invention for providing a feedback data indicating quality of a food processing performed by an operator.

### DESCRIPTION OF EMBODIMENTS

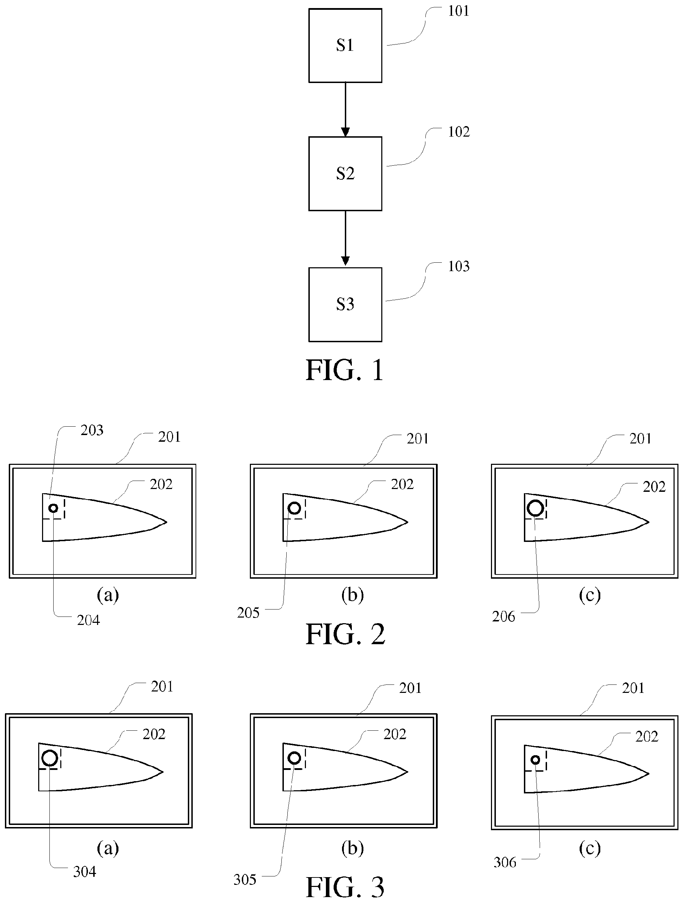

Figure 1 shows a flowchart of a method according to the present invention of providing a feedback data indicating quality of a food processing performed by an operator.

In step (S1) 101, at least one image data is acquired of a processed food product from an operator. This image data may e.g. include X-ray data, 2 and/or 3 Dimensional image data, or any other type of image data acquired after the food product is processed.

The operator may be arranged at a processing station selected from a plurality of processing stations arranged along an infeed conveyor where e.g. via sweep arms, the food product is automatically moved from the infeed conveyor to the processing station where the operator processes the food product, e.g. removes pin bones from a fish fillet and trims the fish fillet. The food product is then automatically conveyed by e.g. a take-away conveyor through e.g. an inspection apparatus, 2D/3D inspection device or any other type of inspection device.

In step (S2) 102, the acquired image data is processed, where the processing includes at least the step of detecting whether undesired objects are present in the processed food product, obtaining, in case undesired objects are detected, position data of the undesired objects within the processed food product.

In step (S3) 103, the position data is utilized in issuing a feedback indicator indicating a position where said detected undesired objects are present within the food product processed by said operator.

Figure 2 illustrates one embodiment of issuing such a feedback indicator as discussed in relation to figure 1, showing a display 201 that may illustrate outlines 202 of the food product being processed, in the example shown here, the food product is a fish fillet. These outlines may be understood as reference outlines as shown here showing roughly an outer shape of a fish fillet. Another alternative (not shown here) would be to illustrate the outlines of the actual food product based on the subsequent image data.

Figures 2a - 2c illustrate an embodiment where said feedback indicator is utilized as a pixel command in determining a pixel illumination area within a pixel array in the display 201.

The position data discussed in relation to figure 1 is 203, i.e. where undesired objects such as bones was detected, may be area 203. The visual presentation is presented by a pixel array circle 204 (or any other shaped form) indicating via the circle shown here that such an undesired object was detected within area 203. In that way, the operator (or plant manager) may immediately see where the quality of the food processing is lacking.

Figure 2b and 2c illustrate an embodiment where undesired objects were detected within the same area 203 in successive food products.

Figure 2b illustrates an example of lack of quality in the food processing present in one or more subsequent food products within the same area 203. As shown here, an intensifier is triggered that in this case magnifies said pixel command that triggers an enlargement of said illumination area within said pixel array. Figure 2c shows a further enlargement due to repetitive lack of qualities in the food processing within area 203.

Figure 3 illustrates similar scenario as depicted in figure 2, but in this embodiment, an improvement is visible via successively decrease in the pixel array circle 304, 305 and 306, where a de-intensifier is triggered issuing a pixel command indicating a successive decrease in the pixel array because of improvement by the operator.

The display shown in figures 2 and 3 may be a display provided said operator's processing station, which may be among other processing stations arranged along an infeed conveyor, where the food products may be delivered to the processing stations automatically via e.g. a sweep arm or the like, and where the processed food products may subsequently be received by a take-away conveyor, and subsequently conveyed through a detection apparatus, e.g. a digital camera, X-ray apparatus or similar devices well known to a person skilled in the art.

The display 201 may also be arranged at a plant manager that allows the plant manager to monitor the quality of the processing so as to evaluate for example training for individual operators.

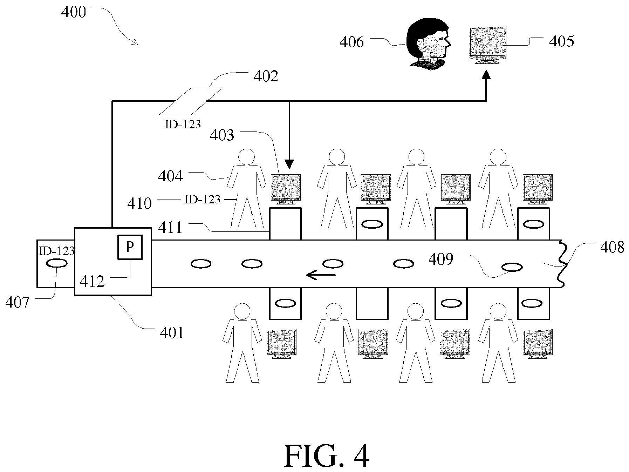

Figure 4 shows an embodiment of a system 400 according to the present invention for providing a feedback data indicating quality of a food processing performed by an operator 404, or as shown here is selected from a plurality of operators, stationed along a food processing line comprising an infeed conveyor 408 and an outfeed conveyor, which as shown in this embodiment is the same conveyor as the infeed conveyor.

In operation, incoming food products 409 are conveyed and automatically assigned to a selected processing station available, where the food product is processed. The processing may e.g. include among other things removing fat, undesired tissues, cartilage and bones. After processing the food products, they are placed back on the same conveyor (typically in an automatic way) such that they do not overlap with food products on the conveyor 408 (another takeaway conveyor may also be used, being different from the infeed conveyor).

As an example, an operator 404 who has logged in to a processing station 411 via an unique Identification Code "ID-123" 410 has processed a food product 407.

In the embodiment shown here, the food product 407 has the ID-123 associated to it via tracking the food product as it passed through a detection apparatus, e.g. an X-ray apparatus or any type of detection mechanism used in the food industry.

The image data are processed by a processor 412, e.g. a processor comprised in the detection apparatus 401, where the processing comprises detecting whether undesired objects are present in the processed food product. If undesired objects are detected, the position where the undesired objects are present within the processed food product is determined, similar as discussed in relation to figures 1 - 3.

The processor is further configured to utilize the position data along with the associated ID data in issuing a feedback indicator 402 indicating a position where said detected undesired objects are present within the food product processed by said operator.

The feedback indicator may then, similar as discussed in relation to figures 2 and 3, be utilized in visually displaying on a display 403 associated with the operator 404 where the quality of the processing was lacking and/or this data may be presented to a plant manager 406 on a display 405 where improvements are needed. Based on this information, the plant manager 406 has an overview over how the different operators with different ID's are performing and react on that by e.g. additional training.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention as defined in the claims, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage.

## Claims

1. A method of providing a feedback data indicating quality of food processing performed by an operator, comprising:
• acquiring at least one image data (101) of a processed food product (407) from an operator,
• processing the acquired image data (102), the processing including:
o detecting whether undesired objects are present in the processed food product,
o obtaining, in case undesired objects are detected, position data (203) of the undesired objects within the processed food product,
• utilizing the position data (103) in issuing a feedback indicator (204) indicating a position where said detected undesired objects are present within the food product processed by said operator,
**characterized in that** in case undesired objects are detected in a subsequent processed food product processed by said operator, where the undesired objects are positioned within the same or similar position as for said processed food product, an intensifier is triggered indicating an increase in magnitude of said feedback indicator.

2. The method according to claim 1, wherein said feedback data is visually shown to the operator via a display (201).

3. The method according to claim 2, wherein visually showing said feedback data comprises showing said feedback data within outlines of a food product being processed.

4. The method according to any of the preceding claims, wherein said feedback indicator is utilized as a pixel command in determining a pixel illumination area within a pixel array.

5. The method according to claim 4, wherein said intensifier is utilized as a magnification of said pixel command indicating an enlargement of said illumination area within said pixel array.

6. The method according to any of the claims 4 or 5, wherein said illumination areas are indicated within a reference contour profile (202) indicating the contour of said food products.

7. The method according to any of the preceding claims, wherein in case no undesired objects are detected within the same or similar position as for said processed food product in a subsequent processed food product processed by said operator, a de- intensifier is triggered indicating a decrement of said feedback.

8. The method according to claim 7 and claim 4, wherein said de-intensifier is utilized as a de-magnification of said pixel command indicating a decrement of said illumination area within said pixel array.

9. The method according to any of the preceding claims, wherein said at least one acquired image data comprises one or more of the following:
X-ray data acquired from an X-ray apparatus,
2 Dimensional (2D) digital data acquired from a 2D digital device,
3D digital data acquired form a 3D digital device.

10. The method according to any of the preceding claims, further comprising associating Identification Data (ID) of said operator to said feedback indicator.

11. An automatic feedback system (400) configured to provide a feedback data indicating quality of a food processing performed by an operator, comprising:
• a detection apparatus (401) for acquiring image data of a processed food product (407) from an operator (404),
• a processor (412) for processing the acquired image data, the processing including:
∘ detecting whether undesired objects are present in the processed food product,
∘ determining, in case undesired objects are detected, the position of the undesired objects within the processed food product, wherein the processor is further configured to utilize the position data in issuing a feedback indicator (402) indicating a position (203) where said detected undesired objects (204) are present within the food product processed by said operator,
**characterized in that** the processor is configured to identify a subsequent erroneously processed food product processed by said operator, wherein the subsequent erroneously processed food product is a food product with an undesired objects positioned within the same or similar position as for said processed food product.

12. The automatic feedback system according to claim 11, wherein the processor is configured to generate an intensifier signal in response to the identification of the subsequent erroneously processed food product, the intensifier signal identifying that a subsequent erroneously processed food product was identified.

13. The automatic feedback system according to any of claims 11-12, wherein the processor is configured to determine a distance between a food product with an undesired object and a subsequent erroneously processed food product.

14. The automatic feedback system according to claim 13, wherein the processor is configured to determine the distance in a number of separating food products, a separating food product being a food product arranged in a row of food products between the food products with an undesired object and the subsequent erroneously processed food product.

15. The automatic feedback system according to claim 13, wherein the processor is configured to determine the distance in the form of a time interval separating the food product with an undesired object and the subsequent erroneously processed food product.

16. The automatic feedback system according to any of claims 11-15, further comprising a feedback device associated to the operator, where the feedback device is operable connected to the processor and comprises a display, where said feedback indicator is utilized as a pixel command in determining a pixel illumination area within the display.

17. The automatic feedback system according to claim 16, wherein the intensifier signal is constituted by a change in intensity of the pixel illumination, or a change in size of the illuminated area, or a change in color of the illuminated area.

18. The automatic feedback system according to any of claims 11 - 17, wherein said detection apparatus comprises an X-ray apparatus and wherein said image data comprises X-ray data, and/or digital imaging device for acquiring 2 Dimensional and/or 3 Dimensional image data of the processed food product.

19. The automatic feedback system according to any of the claims 11 - 18, wherein said food product is processed at a processing station selected from a plurality of processing stations arranged along an infeed conveyor from which the food product is received, and wherein after processing said food product, it is received by a take-away conveyor and conveyed to said detection apparatus where said image data is acquired.

## Patentansprüche

1. Verfahren zur Bereitstellung von Rückmeldungsdaten, welche die Qualität der Nahrungsmittelverarbeitung, die durch einen Bediener durchgeführt wird, angeben, welches Folgendes umfasst:
• Erfassen von mindestens einem Satz Bilddaten (101) eines verarbeiteten Nahrungsmittelproduktes (407) von einem Bediener,
• Verarbeiten der erfassten Bilddaten (102), wobei das Verarbeiten Folgendes umfasst:
∘ Erkennen, ob unerwünschte Objekte in dem verarbeiteten Nahrungsmittelprodukt vorliegen,
∘ Erhalten, falls unerwünschte Objekte erkannt werden, von Positionsdaten (203) der unerwünschten Objekte innerhalb des verarbeiteten Nahrungsmittelproduktes,
• Nutzen der Positionsdaten (103) beim Ausgeben eines Rückmeldungsindikators (204), der eine Position, an welcher die erkannten unerwünschten Objekte innerhalb des Nahrungsmittelproduktes, das durch den Bediener verarbeitet wird, vorliegen, angibt,
**dadurch gekennzeichnet, dass**, falls unerwünschte Objekte in einem anschließenden verarbeiteten Nahrungsmittelprodukt, das durch den Bediener verarbeitet wird, erkannt werden, wobei sich die unerwünschten Objekte innerhalb der gleichen oder einer ähnlichen Position wie bei dem verarbeiteten Nahrungsmittelprodukt befinden, ein Verstärker ausgelöst wird, welcher eine Zunahme in der Größenordnung des Rückmeldungsindikators angibt.

2. Verfahren nach Anspruch 1, wobei die Rückmeldungsdaten dem Bediener visuell über eine Anzeige (201) gezeigt werden.

3. Verfahren nach Anspruch 2, wobei das visuelle Zeigen der Rückmeldungsdaten das Zeigen der Rückmeldungsdaten innerhalb von Umrissen eines gerade verarbeiteten Nahrungsmittelproduktes umfasst.

4. Verfahren nach einem der vorhergehenden Ansprüche, wobei der Rückmeldungsindikator als ein Pixelbefehl beim Bestimmen eines Pixelbeleuchtungsbereiches innerhalb einer Pixelanordnung genutzt wird.

5. Verfahren nach Anspruch 4, wobei der Verstärker als eine Vergrößerung des Pixelbefehls genutzt wird, welche eine Erweiterung des Beleuchtungsbereiches innerhalb der Pixelanordnung angibt.

6. Verfahren nach einem der Ansprüche 4 oder 5, wobei die Beleuchtungsbereiche innerhalb eines Referenzkonturprofils (202), welches die Kontur der Nahrungsmittelprodukte angibt, angegeben werden.

7. Verfahren nach einem der vorhergehenden Ansprüche, wobei, falls keine unerwünschten Objekte innerhalb der gleichen oder einer ähnlichen Position wie bei dem verarbeiteten Nahrungsmittelprodukt bei einem anschließenden verarbeiteten Nahrungsmittelprodukt, das durch den Bediener verarbeitet wird, erkannt werden, ein Abschwächer ausgelöst wird, welcher eine Verminderung der Rückmeldung angibt.

8. Verfahren nach Anspruch 7 und Anspruch 4, wobei der Abschwächer als eine Verkleinerung des Pixelbefehls genutzt wird, welche eine Verminderung des Beleuchtungsbereiches innerhalb der Pixelanordnung angibt.

9. Verfahren nach einem der vorhergehenden Ansprüche, wobei der mindestens eine erfasste Satz Bilddaten eines oder mehrere der Folgenden umfasst:
Röntgendaten, die durch eine Röntgenvorrichtung erfasst werden,
zweidimensionale (2D) Digitaldaten, die von einem 2D-Digitalgerät erfasst werden, und
3D-Digitaldaten, die von einem 3D-Digitalgerät erfasst werden.

10. Verfahren nach einem der vorhergehenden Ansprüche, welches ferner das Assoziieren von Identifikationsdaten (ID) des Bedieners mit dem Rückmeldungsindikator umfasst.

11. Automatisches Rückmeldungssystem (400), das konfiguriert ist zum Bereitstellen von Rückmeldungsdaten, welche die Qualität einer Nahrungsmittelverarbeitung, die durch einen Bediener durchgeführt wird, angeben, welches Folgendes umfasst:
• eine Erkennungsvorrichtung (401) zum Erfassen von Bilddaten eines verarbeiteten Nahrungsmittelproduktes (407) von einem Bediener (404)
• einen Prozessor (412) zum Verarbeiten der erfassten Bilddaten, wobei das Verarbeiten Folgendes umfasst:
∘ Erkennen, ob unerwünschte Objekte in dem verarbeiteten Nahrungsmittelprodukt vorliegen,
∘ Bestimmen, falls unerwünschte Objekte erkannt werden, der Position der unerwünschten Objekte innerhalb des verarbeiteten Nahrungsmittelproduktes,
wobei der Prozessor ferner konfiguriert ist zum Nutzen der Positionsdaten beim Ausgeben eines Rückmeldungsindikators (402), welcher eine Position (203) angibt, an welcher die erkannten unerwünschten Objekte (204) innerhalb des Nahrungsmittelproduktes, das durch den Bediener verarbeitet wird, vorliegen, **dadurch gekennzeichnet, dass** der Prozessor konfiguriert ist zum Identifizieren eines anschließenden fehlerhaft verarbeiteten Nahrungsmittelproduktes, das durch den Bediener verarbeitet wird, wobei es sich bei dem anschließenden fehlerhaft verarbeiteten Nahrungsmittelprodukt um ein Nahrungsmittelprodukt mit unerwünschten Objekten an einer Position innerhalb der gleichen oder einer ähnlichen Position wie bei dem verarbeiteten Nahrungsmittelprodukt handelt.

12. Automatisches Rückmeldungssystem nach Anspruch 11, wobei der Prozessor konfiguriert ist zum Erzeugen eines Verstärkersignals als Reaktion auf die Identifikation des anschließenden fehlerhaft verarbeiteten Nahrungsmittelproduktes, wobei das Verstärkersignal identifiziert, dass ein anschließendes fehlerhaft verarbeitetes Nahrungsmittelprodukt identifiziert wurde.

13. Automatisches Rückmeldungssystem nach einem der Ansprüche 11 - 12, wobei der Prozessor konfiguriert ist zum Bestimmen einer Distanz zwischen einem Nahrungsmittelprodukt mit einem unerwünschten Objekt und einem anschließenden fehlerhaft verarbeiteten Nahrungsmittelprodukt.

14. Automatisches Rückmeldungssystem nach Anspruch 13, wobei der Prozessor konfiguriert ist zum Bestimmen der Distanz in einer Reihe trennender Nahrungsmittelprodukte, wobei es sich bei einem trennenden Nahrungsmittelprodukt um ein Nahrungsmittelprodukt handelt, das in einer Reihe von Nahrungsmittelprodukten zwischen den Nahrungsmittelprodukten mit einem unerwünschten Objekt und dem anschließenden fehlerhaft verarbeiteten Nahrungsmittelprodukt angeordnet ist.

15. Automatisches Rückmeldungssystem nach Anspruch 13, wobei der Prozessor konfiguriert ist zum Bestimmen der Distanz in Form eines Zeitintervalls, welches das Nahrungsmittelprodukt mit einem unerwünschten Objekt und das anschließende fehlerhaft verarbeitete Nahrungsmittelprodukt trennt.

16. Automatisches Rückmeldungssystem nach einem der Ansprüche 11 - 15, welches ferner ein Rückmeldungsgerät umfasst, das mit dem Bediener assoziiert ist, wobei das Rückmeldungsgerät wirkungsmäßig mit dem Prozessor verbunden ist und eine Anzeige umfasst, wobei der Rückmeldungsindikator als ein Pixelbefehl beim Bestimmen eines Pixelbeleuchtungsbereiches innerhalb der Anzeige genutzt wird.

17. Automatisches Rückmeldungssystem nach Anspruch 16, wobei das Verstärkersignal durch eine Änderung in der Intensität der Pixelbeleuchtung oder eine Änderung in der Größe des beleuchteten Bereiches oder eine Änderung in der Farbe des beleuchteten Bereiches gebildet wird.

18. Automatisches Rückmeldungssystem nach einem der Ansprüche 11 - 17, wobei die Erkennungsvorrichtung eine Röntgenvorrichtung umfasst, wobei die Bilddaten Röntgendaten umfassen, und/oder ein digitales Bildgebungsgerät zum Erfassen von zweidimensionalen und/oder dreidimensionalen Bilddaten des verarbeiteten Nahrungsmittelproduktes.

19. Automatisches Rückmeldungssystem nach einem der Ansprüche 11 - 18, wobei das Nahrungsmittelprodukt an einer Verarbeitungsstation ausgewählt aus mehreren Verarbeitungsstationen, die entlang eines Zuführförderers, von welchem das Nahrungsmittelprodukt empfangen wird, angeordnet sind, verarbeitet wird und wobei das Nahrungsmittelprodukt, nach seiner Verarbeitung, durch einen Abnahmeförderer empfangen und zu der Erkennungsvorrichtung transportiert wird, an welcher die Bilddaten erfasst werden.

## Revendications

1. Procédé de fourniture d'une donnée de rétroaction indiquant la qualité d'un traitement alimentaire réalisé par un opérateur, comprenant :
• l'acquisition d'au moins une donnée d'image (101) d'un produit alimentaire traité (407) par un opérateur,
• le traitement des données d'image acquises (102), ce traitement incluant :
∘ la détection si des objets non désirés sont présents dans le produit alimentaire traité,
∘ l'obtention, dans le cas où des objets non désirés sont détectés, de données de position (203) des objets non désirés dans le produit alimentaire traité,
• l'utilisation des données de position (103) en éditant un indicateur de rétroaction (204) indiquant une position où lesdits objets non désirés détectés sont présents dans le produit alimentaire traité par ledit opérateur,
**caractérisé en ce que**, dans le cas où des objets non désirés sont détectés dans un produit alimentaire traité suivant traité par ledit opérateur, les objets non désirés étant positionnés dans la même position ou une position similaire à celle dudit produit alimentaire traité, il est déclenché un amplificateur indiquant une augmentation d'amplitude dudit indicateur de rétroaction.

2. Procédé selon la revendication 1, dans lequel ladite donnée de rétroaction est présentée visuellement à l'opérateur par l'intermédiaire d'un afficheur (201).

3. Procédé selon la revendication 2, dans lequel la présentation visuelle de ladite donnée de rétroaction comprend la présentation de ladite donnée de rétroaction dans les contours d'un produit alimentaire en cours de traitement.

4. Procédé selon l'une quelconque des revendications précédentes, dans lequel ledit indicateur de rétroaction est utilisé comme commande de pixels dans la détermination d'une zone d'éclairage à pixels dans une matrice de pixels.

5. Procédé selon la revendication 4, dans lequel ledit amplificateur est utilisé comme système d'agrandissement de ladite commande de pixels indiquant un agrandissement de ladite zone d'éclairage dans ladite matrice de pixels.

6. Procédé selon l'une quelconque des revendications 4 ou 5, dans lequel lesdites zones d'éclairage sont indiquées dans un profil de contour de référence (202) indiquant le contour desdits produits alimentaires.

7. Procédé selon l'une quelconque des revendications précédentes, dans lequel, dans le cas où des objets non désirés sont détectés dans la même position ou une position similaire à celle dudit produit alimentaire traité dans un produit alimentaire traité suivant traité par ledit opérateur, il est déclenché un désamplificateur indiquant une décrémentation de ladite rétroaction.

8. Procédé selon la revendication 7 et la revendications 4, dans lequel ledit désamplificateur est utilisé comme système de désagrandissement de ladite commande de pixels indiquant une décrémentation de ladite zone d'éclairage dans ladite matrice de pixels.

9. Procédé selon l'une quelconque des revendications précédentes, dans lequel ladite au moins une donnée d'image acquise comprend un ou plusieurs des éléments suivants :
des données radiographiques acquises par un appareil radiographique,
des données numériques à 2 dimensions (2D) acquises par un appareil numérique 2D,
des données numériques 3D acquises par un appareil numérique 3D.

10. Procédé selon l'une quelconque des revendications précédentes, comprenant en outre l'association de données d'identification (ID) dudit opérateur audit indicateur de rétroaction.

11. Système de rétroaction automatique (400) configuré pour fournir une donnée de rétroaction indiquant la qualité d'un traitement alimentaire réalisé par un opérateur, comprenant :
• un appareil de détection (401) pour acquérir une donnée d'image (d'un produit alimentaire traité (407) par un opérateur (404),
• un processeur (412) pour traiter les données d'image acquises, ce traitement incluant :
∘ la détection si des objets non désirés sont présents dans le produit alimentaire traité,
∘ la détermination, dans le cas où des objets non désirés sont détectés, de la position des objets non désirés dans le produit alimentaire traité,
le processeur étant en outre configuré pour utiliser les données de position en éditant un indicateur de rétroaction (402) indiquant une position (203) où lesdits objets non désirés détectés (204) sont présents dans le produit alimentaire traité par ledit opérateur, **caractérisé en ce que** le processeur est configuré pour identifier un produit alimentaire suivant erronément traité par ledit opérateur, le produit alimentaire suivant erronément traité étant un produit alimentaire comportant des objets non désirés positionnés à la même position ou une position similaire à celle dudit produit alimentaire traité.

12. Système de rétroaction automatique selon la revendication 11, dans lequel le processeur est configuré pour générer un signal d'intensification en réaction à l'identification du produit alimentaire suivant erronément traité, le signal d'intensification identifiant qu'un produit alimentaire erronément traité a été identifié.

13. Système de rétroaction automatique selon l'une quelconque des revendications 11 à 12, dans lequel le processeur est configuré pour déterminer une distance entre un produit alimentaire comportant un objet non désiré et un produit alimentaire suivant erronément traité.

14. Système de rétroaction automatique selon la revendication 13, dans lequel le processeur est configuré pour déterminer la distance dans un certain nombre de produits alimentaires séparateurs, un produit alimentaire séparateur étant un produit alimentaire disposé dans une rangée de produits alimentaires entre les produits alimentaires comportant un objet non désiré et le produit alimentaire suivant erronément traité.

15. Système de rétroaction automatique selon la revendication 13, dans lequel le processeur est configuré pour déterminer la distance sous la forme d'un laps de temps séparant le produit alimentaire comportant un objet non désiré et le produit alimentaire suivant erronément traité.

16. Système de rétroaction automatique selon l'une quelconque des revendications 11 à 15, comprenant en outre un dispositif de rétroaction associé à l'opérateur, le dispositif de rétroaction étant actionnable en connexion avec le processeur et comprenant un afficheur, ledit indicateur de rétroaction étant utilisé comme commande de pixels dans la détermination d'une zone d'éclairage à pixels dans l'afficheur.

17. Système de rétroaction automatique selon la revendication 16, dans lequel le signal d'intensification est constitué par un changement d'intensité de l'éclairage à pixels, ou un changement de taille de la zone éclairée, ou un changement de couleur de la zone éclairée.

18. Système de rétroaction automatique selon l'une quelconque des revendications 11 à 17, dans lequel ledit appareil de détection comprend un appareil radiographique et ladite donnée d'image comprend des données radiographiques et/ou un dispositif d'imagerie numérique pour acquérir des données d'image à 2 dimensions et/ou à 3 dimensions du produit alimentaire traité.

19. Système de rétroaction automatique selon l'une quelconque des revendications 11 à 18, dans lequel ledit produit alimentaire est traité à un poste de traitement sélectionné parmi une pluralité de postes de traitement disposés le long d'un convoyeur d'alimentation en provenance duquel le produit alimentaire est reçu, et, après le traitement dudit produit alimentaire, il est reçu par un convoyeur d'évacuation et convoyé vers ledit appareil de détection où lesdites données d'image sont acquises.
